# EUROPEAN PATENT APPLICATION

(11) **EP 2 347 740 A1**
(43) Date of publication of application: **27.07.2011**
(21) Application number: 10151589.8
(22) Date of filing: 25.01.2010
(51) Int. Cl.: A61F 9/01

(54) **System and method for performing a presbyopic correction**

(71) Applicant: Technolas Perfect Vision GmbH, 80992 München (DE)
(72) Inventor: Hohla, Kristian, 80799 München (DE); Loesel, Frieder, 68165 Mannheim (DE); Youssefi, Gerhard, Dr., 84028 Landshut (DE)
(74) Representative: Schmidt, Josef Heinrich

(57) **Abstract**

The system according to the present invention is used to create an ablation such that the surface of the cornea comprises a central region having a convex central steepening with a first radius of curvature and a surrounding region having a convex shape with a second radius of curvature, wherein the first radius of curvature is smaller than the second radius of curvature for performing a presbyopic correction. The ablation depth in the central region varies between a minimum ablation depth in the center and a maximum ablation depth at the border of the central region. The ablation depth in the surrounding region is the same as the maximum ablation depth at the border of the central region.

## Description

### Field of Invention

The invention relates to a system and a method for performing a presbyopic correction using a laser system for photoablating tissue of a cornea, preferably using LASIK.

### Background of the Invention

Known procedures for vision correction like PRK, LASIK, and LASEK are typically used to correct ametropia, i.e. myopic and hyperopic vision errors with or without astigmatism by utilizing a volumetric ablation. In customized treatments also higher order aberrations of the eye can be addressed. These procedures are also used for performing a presbyopic correction either alone or in combination with the correction of ametropia.

US 5,533,997 relates to an apparatus and method for performing presbyopia corrective surgery. This known process comprises the steps of anesthetizing a patient, resecting a corneal flap of an eye of the patient to expose a cornea stroma, ablating an annular portion of the corneal stroma using radiation from a laser beam and repositioning the corneal flap onto the eye. During the ablation step a central optic zone of the corneal stroma is left unablated. Thus, by this process a central corneal curvature change is induced to thereby correct presbyopia in the patient. More specifically, an annular ablation of a predetermined width and depth is provided around a central area of the cornea to produce a central protrusion of the stroma. In an embodiment the annular ablation is made on the stroma having a diameter not exceeding 3.5 mm with a central zone varying between 2 and 3 mm. When the corneal flap is repositioned at its initial position, the stromal curvature change is transmitted to the forward corneal surface, thereby transforming it into a multifocal surface, which is in fact myopic in its central part. This document further states that the annular ablation can be made in isolated form, for presbyopia correction, or it can be made together with hyperopia, myopia and astigmatism surgery, either isolated or combined.

US 2009/0234336 A1 relates to methods and systems for treating presbyopia involving ablating a corneal surface of a first and a second eye of a patient. Ablating of a corneal surface of a first eye is performed to enhance vision of near objects through a central zone of the first eye and ablating a corneal surface of a second eye is performed to enhance vision of near objects through a peripheral zone of the second eye. With this method, an optical surface shape shall be established that mitigates or treats presbyopia in a particular patient. The combination of distance vision and near vision in a patient shall be improved on the basis of input patient parameters such as pupil size, residual accommodation, and power need. It is suggested that the power add of the inner region provides a myopic effect to add near vision by bringing the near vision focus closer to the retina. On the other hand, the outer region shall remain unaltered for a distance vision. In order to provide the power add of the inner region an annular ablation is provided around the inner region. It is further stated that the prescriptive refractive ablation shape can have fairly abrupt changes, but post ablation topographies may show that healing of the eye can smooth the transitions. The shape can be applied in addition to any additionally required refractive correction by superimposing the shape on a refractive corrective ablation shape.

### Summary

It is an object of the present invention to provide an improved system and method for performing a presbyopic correction.

This object is achieved by the features of the claims.

An aspect of the invention is directed to a system for creating a corneal shape comprising a laser system for photoablating tissue of a cornea, preferably using LASIK, and a control means for directing a laser beam of the laser system to the corneal tissue. The control means is adapted to create an ablation such that the surface of the cornea comprises a central region having a convex central steepening with a first radius of curvature and a surrounding region having a convex shape with a second radius of curvature, wherein the first radius of curvature is smaller than the second radius of curvature for performing a presbyopic correction. The ablation depth in the central region varies between a minimum ablation depth in the centre and a maximum ablation depth at the border of the central region and the ablation depth in the surrounding region is the same as the maximum ablation depth at the border of the central region.

The ablation can also be performed using surface ablation (PRK) mode or any other way to ablate a predetermined ablation profile.

The basic concept of the invention is to provide a modified shape of the cornea wherein the central region comprises a central steepening with a predetermined first curvature which provides a presbyopic correction and wherein a layer having a substantially uniform thickness is ablated at the surrounding region such that the surrounding region has substantially the same curvature as before the ablation.

According to a further aspect the control means is adapted to create a central steepening, wherein the maximum ablation depth at the border of the central region is in the range of 5 to 15 µm, preferably 7 to 13 µm and most preferable amounts to 10 µm.

The control means is adapted to create the central steepening in the central region having a first diameter in the range of 2.5 to 3.5 mm, preferably 2.8 to 3.2 mm and more preferably 3 mm. The surrounding region has a diameter which is preferably greater than the outer border of the pupil when dilated under dim light condition. Preferably, the surrounding region has a second diameter in the range of 6 to 8 mm, preferably 6.5 to 7.5 mm and more preferably 7 mm.

According to a further aspect of the invention, the system comprises means for calculating the size and/or shape of the central steepening. This calculation is made on the basis of diagnostic data of a patient's eye. The diagnostic data may be subjective data which are obtained by a refractive examination of the patient using a phoropter or other examinations which include the subjective response of the patient to obtain a refractive parameter, especially night vision tests or a test to measure the accommodation range of the patient's eyes. Further subjective data could be subjective accommodation amplitude information based on reading capabilities of the subject at different distances.

The diagnostic data may also be objective data which can be obtained using diagnostic systems which do not involve the subjective response of the patient, such as autorefractometers, pupilometers which deliver for example the size of the mesopic pupil of an eye, wavefront sensors which provide aberration data of an eye, particularly the spherical aberration of the examined eye. Further objective diagnostic exams could be objective measurement of the accommodation amplitude using wavefront sensing, corneal parameters such as asphericity and steepness provided by topography systems and exams which provide the corneal thickness and the thickness of the created flap for example given by ultrasound pachymeters, slit-scanning pachymeters or OCT systems which can be even used intraoperatively to obtain the mentioned flap thickness data.

The means for calculating the size and/or shape of the central steepening preferably calculate the variation of the ablation depth to create said central steepening and/or the maximum ablation depth at the border of the central area. The calculating means preferably further calculate the first diameter of the central region in addition to the before mentioned variation of the ablation depth and/or the maximum ablation depth.

The calculating means further calculate the ablation depth of the region surrounding the central region which is substantially identical to the maximum ablation depth at the border of the central region. The calculating means further calculate the second diameter of the surrounding region in addition to the ablation depth on the basis of diagnostic data of the eye.

According to an embodiment of the invention, the second radius of curvature of the surrounding region is adjusted on the basis of pre-operative and pre-ablative diagnostic data such that the ablation at the surrounding region is refractive neutral. Thus, the ablation at the surrounding region shall have no effect on the optical properties or optical characteristics of the respective portion of the eye.

In another embodiment, the second radius of curvature of the surrounding region is adjusted from the above refractive neutral curvature on the basis of pre-operative and pre-ablative diagnostic data to provide an aspheric correction. Thus, the ablation at the surrounding region results in a change of the optical properties or optical characteristics of said part of the eye.

In a still further embodiment, the second radius of curvature of the surrounding region corresponds to the mean curvature radius based on processed pre-operative diagnostic data of the surrounding region. Preferably, a local curvature of at least part of the surrounding region deviates from the mean curvature radius and said deviation corresponds to the pre-operative diagnostic corneal aberration data of the corneal topography in said surrounding region. As an alternative, a local curvature of at least part of the surrounding region deviates from the mean curvature radius and said deviation corresponds to the pre-operative diagnostic ocular wavefront aberration data of the whole eye in said surrounding region.

The presbyopic correction can be combined to basically the total range of indications covered with the excimer laser. This can be achieved simply by combining the new ablation profile to any classical treatment profile for the correction of ametropia, for example myopia, hyperopia both either alone or in combination with astigmatism.

According to an aspect of the present invention, the new ablation profile which is used to perform a presbyopic correction is used as a standard ablation profile without taking further additional parameters of the eye into account. Such additional parameters of the eye comprise refraction, topography, pachymetry, pupil size information, corneal thickness or flap thickness information, or other corneal shape parameters such as K-Reading and asphericity as long as these are within a certain normal band of the population. The combination with any classical treatment profile for the correction of ametropia is determined in a separate step.

In the case that the cornea comprises an asphericity, the classical treatment with an excimer laser will correct the ametropia by using an aspheric ablation profile which maintains the corneal asphericity. In the case of an inappropriate pre-operative corneal asphericity, the basic profile for correcting the ametropia will be a profile which adjusts the corneal asphericity to a range in which the shape of the central steepening is most effective in providing a near visual acuity.

According to a modification, the standard ablation profile for performing the presbyopic correction can be combined with a wavefront guided profile to correct the aberrations including higher order aberrations in the central region in which the central steepening is formed.

According to a still further modification, the standard ablation profile for performing presbyopic correction can be combined with a wavefront guided profile to correct the aberrations including higher order aberrations in the total region of the desired optical zone, i.e., the central region and at least part of the surrounding region to provide a best possible distance visual acuity in combination with the optical outcome for the near visual acuity provided by said standard ablation profile.

According to an aspect of the invention, the standard ablation profile for performing a presbyopic correction is combined with a high resolution diagnostic reading to obtain the diagnostic data of a patient's eye. This preferably comprises either the pre-operative corneal shape of the eye and/or the post-operative corneal shape of a sufficient number of cases. In the case of determining the pre-operative corneal shape of the eye for active planning of the ablation profile for performing a presbyopic correction, pre-operative asphericity of the cornea can be determined to fine-tune the ablation profile. Moreover, the existing aberrations in the center of the cornea can be measured for removing them with a laser prior to ablating the cornea of a patient's eye using the standard ablation profile for performing the presbyopic correction. This has the advantage that destructive interaction of severe high order aberrations with an ablation profile for performing the presbyopic correction can be avoided.

When using a database with the data of the post-operative corneal shape of a sufficient number of cases, it is possible to assess the most optimal standard ablation profile for performing presbyopic correction, i.e., preferably the shape and amount of the ablation depth. The corneal shape of this optimal standard ablation profile is preferably constant. Alternatively, it is possible to develop a nomogram how the treatment for a next case should be designed on the basis of the result of collected cases, which are typically saved in a database.

According to another aspect, the means for calculating the size and/or shape of the central steepening create an elliptic central region or any other non-circular central region. In the case of an elliptic central region, the short axis to the long axis of its elliptic area may have a relation of 2 to 3 up to 3 to 4. Said elliptic central region is preferably arranged with its long axis in the same direction as the axis of an astigmatic refractive component of the eye.

The elliptical shape of the central steepening region is adapted to compensate for any pre-operative diagnostic data of the eye. In particular, a subjective refractive error can be compensated. Furthermore, an objective refractive error of the eye can be compensated.

According to another aspect, the system comprises means for receiving diagnostic pre-operative data, wherein said data comprise pre-operative corneal surface data and/or pre-ablative flap thickness data and/or intra-operative eye image data. In an embodiment, a precise pachymetry reading is obtained to assess the thickness of the created flap to provide a base for the appropriate amplitude of the ablation profile for performing a presbyopic correction. As a final cornea steepening is a result of the interaction of the created central steepening in the stromal bed by the photoablation and the coverage of the flap a fine adjustment of said amplitude of the ablation profile can be made. A flap typically has a thickness of 90 to 130 µm. However, the flap thickness may vary and due to a possible influence on the final cornea steepening, it is preferable to either control the thickness of the flap and/or to make a respective adjustment of the amplitude of the ablation profile. In other words, the maximum ablation depth at the border of the central region is adapted according to the thickness of the flap. For example, in the case of a flap thickness of 100 µm, the maximum ablation depth may amount to 10 µm. On the other hand, in the case of a flap thickness of 140 µm, the maximum ablation depth may amount to 14 µm. On the other hand, if the flap thickness amounts to 90 µm, the maximum ablation depth may be 9 µm. Thus, the same intended presbyopic correction may be achieved when taking into account the flap thickness for determining the maximum ablation depth at the border of the central region.

According to a still further aspect of the invention, the system comprises means for receiving diagnostic pre-operative data, which comprise pupil geometry data. The pupil geometry data are used to adjust the first diameter of the central region, in particular with reference to the pupil diameter of the eye. Moreover, the second diameter of the surrounding region may be adjusted according to the maximum pupil diameter of the eye. Preferably, the pupil diameter of a dilated eye under dim light conditions is taken as a reference for adjusting the diameter of the surrounding region.

According to an embodiment of the invention, the relation between the first diameter of the central region and the diameter of the dilated pupil is in the range of 0.50 to 0.63, more preferably in the range of 0.55 to 0.60 and most preferably 0.57. Alternatively, the relationship between the area of the central region and the area of the dilated pupil may amount to 0.25, 0.3 0, 0.3 5 or 0.40.

According to another aspect of the invention, the calculation unit processes intra-operative eye image data to position and to re-adjust the ablation profile in terms of the centration on the apex of the cornea of the eye. In that case, preferably any aberration data which are determined relative to the pupil center in the apex centred system are re-calculated and then combined with the ablation profile for performing a presbyopic correction. During the ablation, an eye tracker may be used for tracking the ablation in x-, y-, and z-dimension and in addition in a rotational position preferably by using the image of the eye.

The system preferably comprises a storage unit which contains historical data on pre-operative diagnostic readings and respective optimised shape and/or size of standard ablation profiles in relation to the achieved post-operative historical data in terms of achieved visual performance for near and distance. These historical data can be used for adjusting the final standard ablation profile which is delivered to the eye. Moreover, it allows to choose between a limited set of different standard ablation profiles according a nomogram proposal derived from the historical data.

### Brief Description of the Drawings

Illustrative, non-limiting embodiments of the present invention will be described by way of example with reference to the accompanying drawings, in which the same reference number is used to designate the same or similar components in different figures.
Figure 1 shows a simplified diagram of a cross section of a pre-operative corneal shape of a patient's eye,
Figure 2 shows a simplified diagram of a post-operative corneal shape of a patient's eye after application of an ablation profile according to an embodiment of the present invention,
Figure 3 shows a diagram with examples of an ablation profile according to an embodiment of the present invention,
Figure 4 shows a simplified diagram of a post-operative corneal shape using the LASIK procedure showing the resected corneal flap,
Figure 5 shows the simplified diagram of the post-operative corneal shape as shown in Fig. 4 after repositioning the flap on the patient's eye and
Figure 6 shows a block diagram of a system according to an embodiment of the invention.

### Detailed Description

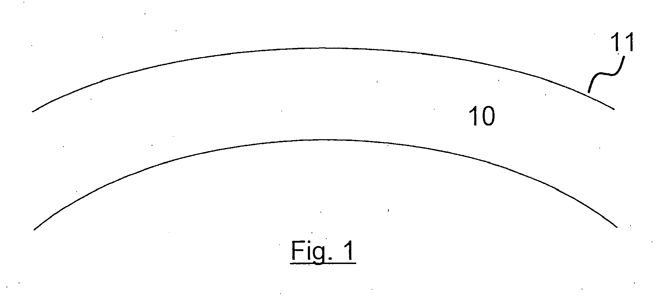

Figure 1 shows a diagram of a cross section of a cornea of a patient's eye, wherein the cornea 10 comprises an anterior surface 11 having a predetermined original curvature. In this diagrammatic view, the opened flap for performing a LASIK procedure is not shown.

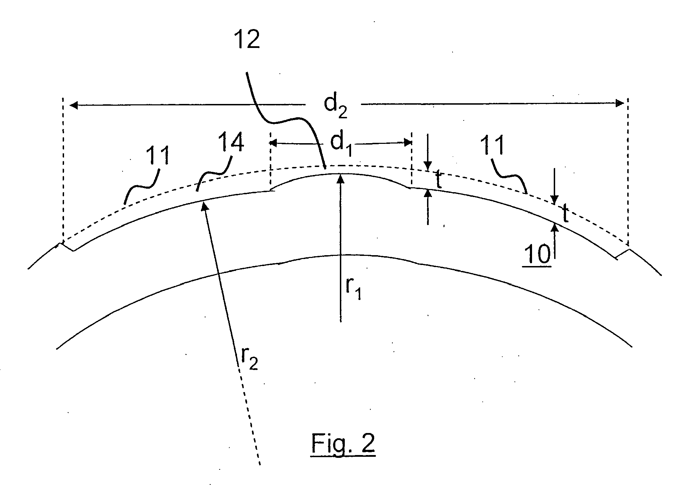

Figure 2 shows a post-operative corneal shape after application of an ablation profile according to an embodiment of the present invention. As shown in this cross section, the cornea 10 comprises a modified outer surface which is formed by photoablation of tissue using a laser system. The original outer surface 11 is also shown as a dashed line. The central region has a convex central steepening with a first radius of curvature r₁ and a surrounding region having a convex shape with a second radius of curvature r₂. As indicated, the first radius of curvature r₁ is smaller than the second radius of curvature r₂. The central region has a diameter d₁. The amount of ablation in this central region varies from the center, where only a small ablation is made, to a maximum ablation at the border of this central region. The maximum ablation is indicated as an ablation depth t. Moreover, a surrounding region 14 is formed by ablating a layer with a constant thickness t, wherein the constant thickness t corresponds to the maximum ablation depth at the border of the central region. The diameter of the surrounding region is indicated with d₂.

The profile obtained after ablation as shown in Figure 2 has the effect that the central steepening 12 is used for performing a presbyopic correction. On the other hand, the surrounding region as a result of ablating a layer of constant thickness does not have any effect on the optical characteristics or optical properties so that this part of the patient's eye is not affected in refraction by the ablation. In other words, the ablation in the surrounding region is refractive neutral.

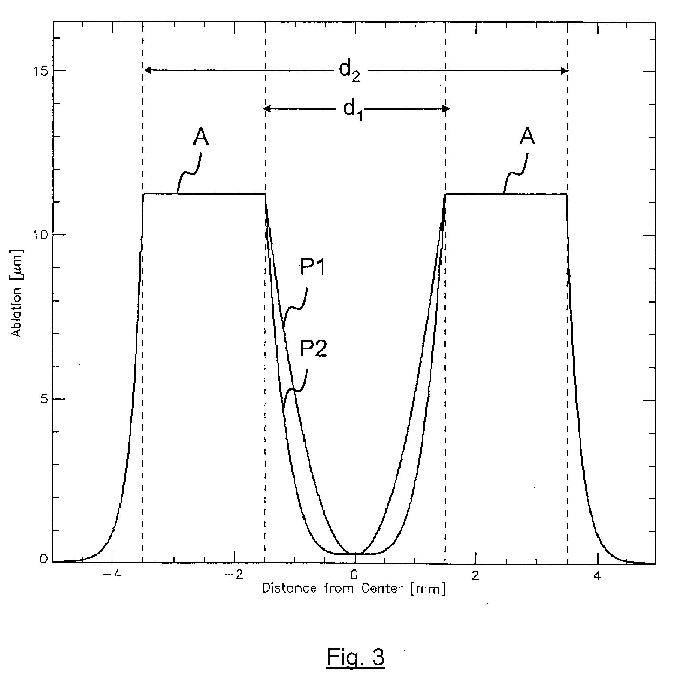

The ablation as shown in Figure 2 may be the result of an ablation profile as principally shown in Figure 3. Figure 3 shows two different ablation profiles P1, P2 which all have the same ablation amount A in a surrounding region and different shapes of the ablation profile in the central region. Within the central region, a first profile P1 corresponds to a curve of second order. A second ablation profile P2 corresponds to a curve of fourth order. However, the ablation profiles could also correspond to curves of other orders, e.g. to a curve of sixth or eighth order. In all cases, the ablation profile within the central region extends over the diameter d₁. Due to the different shape of the ablation profiles P1 and P2, the resulting central steepening will receive a corresponding shape. For all ablation profiles, the amount of ablation varies within the central region from a small amount of ablation at a center of the central region to a large amount of ablation at the border of the central region. As can be seen the variation of the amount of ablation is greater in a central part of the central region for the first ablation profile P1 than for the other ablation profile P2. On the other hand the variation of the amount of ablation at the outer part of the central region is greater for the second ablation profile P2 than for the other ablation profile P1. In all cases, the ablation profile in the central region has a maximum amount of ablation at the border of the central region. In this example, the maximum ablation amounts to about 11 µm, i.e., the corresponding maximum ablation depth at the cornea will amount to about 11 µm. There is a strong discontinuity at the border of the central region as the ablation in the surrounding region is constant from the border of the central region to an outer border of the surrounding region. In this example, this constant ablation amounts to about 11 µm as the maximum ablation depth at the border of the central region. Outside the diameter d₂ of the surrounding region, there is a transition zone in which the ablation amount changes from 11 µm to 0 µm.

In the example shown in Figure 3, the first diameter d₁ amounts to about 3 mm. The second diameter d₂ of the surrounding region amounts to about 7 mm. In this example, the ablation profile is symmetric with respect to the center of the central region. Furthermore, the transition zone in this example has a diameter of about 9 mm.

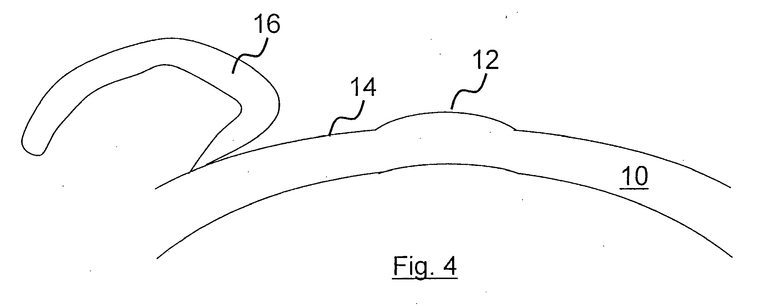

Figure 4 shows a diagram similar to Figure 2 after application of an ablation profile. In particular, a cornea 10 comprises a central corneal steepening 12 after using LASIK procedure. Moreover, the corneal flap 16 is shown in a diagrammatic manner.

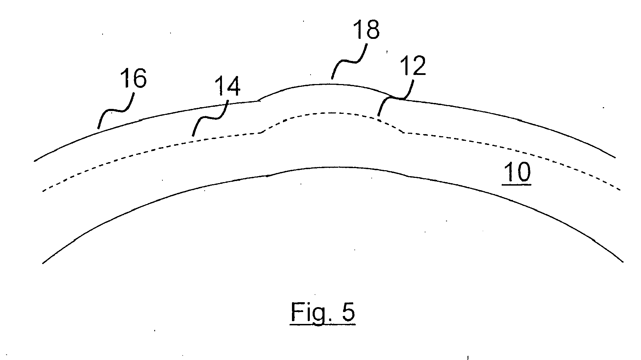

Figure 5 shows the same cornea as in Figure 4 after repositioning the flap onto the cornea. More specifically, Figure 5 shows a cornea 10 with the surrounding region 14 and the corneal steepening 12 before the flap is closed as a dashed line. Moreover, the repositioned flap 16 is shown and the resulting central steepening 18 at the outer surface of the repositioned flap.

In this example, the effect of the flap on the residual amplitude of steepening after the flap is closed is shown. The resulting central steepening 18 has substantially the same curvature as the corneal steepening 12. However, depending on the thickness of the flap, the resulting central steepening 18 may vary.

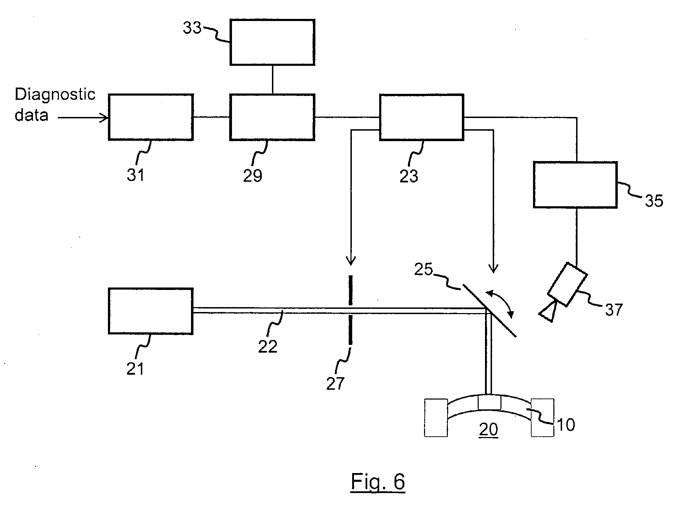

Figure 6 shows a block diagram of an exemplary embodiment of a system according to the present invention. The system for creating a corneal shape comprises a laser system 21, for example an excimer laser system which outputs a laser beam 22 which is suitable for photoablation of corneal tissue 10 of a patient's eye 20. The system further comprises a controller 23 which is connected to a mirror 25 that guides the laser beam 22 from the laser system 21 to the cornea 10 of the eye 20. The controller is further connected to a shutter 27 which is controlled such that during a treatment phase the shutter is open so that the laser beam 22 reaches the mirror 25 and then the cornea 10. During other times the shutter 27 is closed and the laser beam 22 is stopped at the shutter. The system further comprises calculating means 29 for calculating an ablation profile for a presbyopic correction on the basis of pre-operative data and post-operative data of the patient's eye. The calculation means 29 is connected to means 31 which are adapted to receive said pre-operative diagnostic data and post-operative data. The system further comprises a storage unit 33 for storing historical diagnostic pre-operative and post-operative data and corresponding ablation profiles for an optimised shape of a central steepening. The calculation means 29 is connected to the storage unit 33 and it is adapted to select at least one of the stored ablation profiles with reference to received diagnostic pre-operative and post-operative data. In this embodiment an eye tracker 35 is connected to the controller 23. The eye tracker 35 is also connected to a camera 37 which takes images of the patient's eye 20. The eye tracker 35 is used for tracking the ablation in x-, y-, and z-dimension and in addition in a rotational position preferably by using the image of the eye.

The system is used to create an ablation such that the surface of the cornea comprises a central region having a convex central steepening with a first radius of curvature and a surrounding region having a convex shape with a second radius of curvature, wherein the first radius of curvature is smaller than the second radius of curvature for performing a presbyopic correction. The ablation depth in the central region varies between a minimum ablation depth in the center and a maximum ablation depth at the border of the central region. The ablation depth in the surrounding region is the same as the maximum ablation depth at the border of the central region.

While certain embodiments have been chosen to illustrate the invention it will be understood by those skilled in the art that changes and modifications can be made therein without departing from the scope of the invention as defined in the appended claims.

## Claims

1. System for creating a corneal shape comprising:
a laser system for photoablating tissue of a cornea, preferably using LASIK, and a control means for directing a laser beam of the laser system to the corneal tissue, wherein the control means is adapted to create an ablation such that the surface of the cornea comprises a central region having a convex central steepening with a first radius of curvature and a surrounding region having a convex shape with a second radius of curvature, wherein the first radius of curvature is smaller than the second radius of curvature for performing a presbyopic correction, **characterized in that** the ablation depth in the central region varies between a minimum ablation depth in the centre and a maximum ablation depth at the border of the central region and that the ablation depth in the surrounding region is substantially the same as the maximum ablation depth at the border of the central region.

2. System according to claim 1, wherein the control means is adapted to create a central steepening, wherein the maximum ablation depth is in the range of 5 to 15 µm, preferably amounts to 10 µm.

3. System according to claim 1 or 2, wherein the control means is adapted to create the central steepening in the central region having a first diameter in the range of 2.5 to 3.5 mm, preferably 2.8 to 3.2 mm and more preferably 3 mm and wherein the surrounding region has a second diameter in the range of 6 to 8 mm, preferably 6.5 to 7.5 mm and more preferably 7 mm.

4. System according to any of claims I to 3, further comprising means for calculating the size and/or shape of the central steepening, preferably for calculating the variation of the ablation depth to create the central steepening and/or the maximum ablation depth at the border of the central area and/or the first diameter of the central area on the basis of diagnostic data of an eye to perform a presbyopic correction.

5. System according to claim 4, wherein said calculating means further calculate the maximum ablation depth of the surrounding region and/or the second diameter of the surrounding region on the basis of diagnostic data of the eye.

6. System according to claim 5, wherein said second radius of curvature of the surrounding region is adjusted on the basis of pre-operative and pre-ablative diagnostic data to be refractive neutral.

7. System according to claim 5, wherein said second radius of curvature of the surrounding region is adjusted on the basis of pre-operative and pre-ablative diagnostic data to provide an aspheric correction.

8. System according to claim 5, wherein said second radius of curvature of the surrounding region is the mean curvature radius based on processed pre-operative diagnostic data of the surrounding region.

9. System according to claim 8, wherein a local curvature of at least a part of the surrounding region deviates from the mean curvature radius and said deviation corresponds to the pre-operative diagnostic corneal aberration data of the corneal topography in said surrounding region or wherein a local curvature of at least a part of the surrounding region deviates from the mean curvature radius and said deviation corresponds to the pre-operative diagnostic ocular wavefront aberration data of the whole eye in said surrounding region.

10. System according to any of claims of 4 to 9, wherein the means for calculating the size and/or shape of the central steepening creates an elliptic area for the central steepening region.

11. System according to claim 10, wherein the elliptical shape of the central steepening region is adapted to compensate for the pre-operative diagnostic data of the eye, and wherein preferably the elliptical shape of the central steepening region is specifically adapted to compensate for the subjective refractive error of the eye or the objective refractive error of the eye.

12. System according to any of claims 4 to 11, further comprising means for receiving diagnostic pre-operative data, which comprise pre-operative corneal surface data and/or pre-ablative flap thickness data and/or intra-operative eye image data, and wherein preferably the calculation means process pre-operative corneal surface data to combine an aberration corrective treatment with the treatment for correcting presbyopia.

13. System according to any of claims 4 to 12, further comprising means for receiving diagnostic pre-operative data, which comprise pupil geometry data to adjust the diameter of the central region according to the pupil diameter of the eye and/or further comprising means for receiving diagnostic pupil data to adjust the diameter of the surrounding region according to the maximum pupil diameter of the eye.

14. System according to any of claims 4 to 13, wherein said calculation means process pre-ablative flap thickness data to re-adjust the variation of the ablation depth to create the central steepening and/or wherein said calculation means process intra-operative eye image data to determine the position and to adapt the ablation profile for creating the central steepening.

15. System according to any of the foregoing claims, further comprising a storage unit for storing historical diagnostic pre-operative and post-operative data and corresponding ablation profiles for an optimised shape of a central steepening, wherein the calculation means is adapted to select at least one of the stored ablation profiles with reference to received diagnostic pre-operative and post-operative data and wherein preferably the calculation means is adapted to adjust an obtained ablation profile by referring to the stored historical data on pre-operative diagnostic data and the corresponding ablation profiles.

16. Method of creating a corneal shape preferably using a system according to any of the foregoing claims comprising the steps of:
creating an ablation such that the surface of the cornea comprises a central region having a convex central steepening with a first radius of curvature and a surrounding region having a convex shape with a second radius of curvature, wherein the first radius of curvature is smaller than the second radius of curvature for performing a presbyopic correction, **characterized in that** the ablation depth in the central region varies between a minimum ablation depth in the centre and a maximum ablation depth at the border of the central region and that the ablation depth in the surrounding region is substantially the same as the maximum ablation depth at the border of the central region.
